(19) Europäisches Patentamt
European Patent Office
Office européen des brevets

(11) Numéro de publication: **0 130 132**
**A1**

(12) # DEMANDE DE BREVET EUROPEEN

(21) Numéro de dépôt: **84401369.8**

(22) Date de dépôt: **27.06.84**

(51) Int. Cl.[4]: **A 61 K 39/44**
A 61 K 39/395

(30) Priorité: **27.06.83 FR 8310587**

(43) Date de publication de la demande:
**02.01.85 Bulletin 85/1**

(84) Etats contractants désignés:
**AT BE CH DE GB IT LI LU NL SE**

(71) Demandeur: **Etablissement Public dit: CENTRE NATIONAL DE LA RECHERCHE SCIENTIFIQUE (CNRS)**
**15, Quai Anatole France**
**F-75007 Paris(FR)**

(72) Inventeur: **Le Pecq, Jean-Bernard**
**37, Square St Charles**
**F-75012 Paris(FR)**

(72) Inventeur: **Tursz, Thomas**
**35, rue Gazan**
**F-75014 Paris(FR)**

(72) Inventeur: **Wiels, Joelle**
**13, rue Ledion**
**F-75014 Paris(FR)**

(72) Inventeur: **Junqua, Simone**
**49, rue de Chatenay**
**F-92160 Antony(FR)**

(74) Mandataire: **Ayache, Monique et al,**
**CABINET PLASSERAUD 84, rue d'Amsterdam**
**F-75009 Paris(FR)**

(54) **Immunotoxine antitumorale, préparations pharmaceutiques la contenant et son utilisation in vitro.**

(57) L'immunotoxine selon l'invention comprend essentiellement un anticorps, de préférence monoclonal, spécifique du globotriaosylcéramide, ou céramidetrihexosyle, de structure:

Galα(1—>4) Galß (1—>4) Glcß (1—>1) céramide,

ou antigène CTH, associé avec une toxine, dans des conditions permettant l'internalisation de la toxine dans les cellules animales.

Cette immunotoxine est utilisable tant *in vitro* qu'*in vivo* pour la destruction des cellules malignes portant l'antigène CTH, même en des quantités non décelables par les méthodes d'analyse précises connues.

EP 0 130 132 A1

Immunotoxine antitumorale, préparations pharmaceutiques la contenant et son utilisation in vitro.

L'invention concerne des médicaments pour la chimiothérapie, notamment des cancers humains.

Elle a plus particulièrement pour objet un agent mixte du type de ceux connus sous l'appellation d'immunotoxines, dans lequel la spécificité de reconnaissance des cellules, notamment tumorales, est conférée par une immunoglobuline et la cytotoxicité par un agent très hautement cytotoxique, appelé ci-après toxine, tel qu'une toxine d'origine bactérienne ou végétale.

Plus précisement, l'invention a pour objet une immunotoxine présentant une haute spécificité vis-à-vis d'un grand nombre de cellules cancéreuses et une internalisation rapide dans celles-ci, son utilisation pour détruire les cellules tumorales in vitro ainsi que les médicaments comprenant cet agent en tant que principe actif.

Le traitement par chimiothérapie des cancers, notamment humains, est généralement limité par le manque de spécificité des agents utilisés, qui entraîne une toxicité souvent difficile à supporter pour le malade. De nombreux chercheurs ont donc tenté d'obtenir des agents plus spécifiques en préparant des agents mixtes, notamment du type des immunotoxines ; une revue de ces agents est faite dans "Immunotoxins : A new approach to cancer therapy" par E.S. VITETTA et coll. Science 219, 644, 1983.

Malheureusement les succès dans ce domaine ont été limités par deux facteurs principaux, à savoir :

- l'utilisation d'anticorps reconnaissant des antigènes non spécifiques des cellules tumorales à traiter ; et
- l'internalisation cellulaire très lente (16 à 30 heures) des immunotoxines, dans ceux des essais qui ont déjà été décrits dans la littérature.

Il convient à ce propos de noter que les démonstrations d'activité ont jusqu'à présent été faites essentiellement in vitro.

L'invention a pour but de remédier, au moins en partie, à ces inconvénients en fournissant une immunotoxine présentant un pouvoir discriminant particulièrement important entre des cellules malignes et des cellules saines et une internalisation rapide de la toxine, dans les seules cellules malignes avec une bonne activité *in vivo*.

Plus précisément, l'immunotoxine selon l'invention est caractérisée en ce qu'elle comprend essentiellement un anticorps, de préférence monoclonal, spécifique du globotriaosylcéramide, ou céramidetrihexosyle, de structure :

Galα(1 → 4)Galβ(1 → 4)Glcβ(1 → 1) céramide,

associé avec une toxine, dans des conditions permettant l'internalisation de la toxine dans les cellules animales.

On sait qu'un certain nombre de cellules cancéreuses, telles qu'entre autres certaines cellules de BURKITT, possèdent à leur surface un glycolipide neutre ayant la même structure que la substance Pk, précurseur de l'antigène du groupe sanguin P ; ce glycolipide est le globotriaosylcéramide ou céramidetrihexosyle appelé ci-après CTH [E. Nudelman et coll., Science, 220, page 508-511, (1983)].

Or, selon l'invention on a constaté qu'une immunotoxine comprenant un anticorps monoclonal spécifique du CTH associé à une toxine, dans des conditions permettant l'internalisation de la toxine dans les cellules animales, internalisait la toxine dans les cellules possédant à leur surface du CTH beaucoup plus rapidement que ne le faisaient les différentes immunotoxines préparées jusqu'à présent et que l'activité thérapeutique d'une telle immunotoxine était supérieure à celle des immunotoxines antérieures.

Ces deux constatations sont déjà en elles-mêmes hautement surprenantes et répondent au but de l'invention, mais ce qui est encore beaucoup plus surprenant et rend

vraisemblablement compte d'un mécanisme d'action différent de celui des immunotoxines de l'art antérieur, c'est que cette immunotoxine est capable de tuer non seulement les cellules portant l'antigène spécifique en grandes quantités (comme cela paraît être le cas pour les cellules du lymphosarcome de BURKITT) mais également toute une série de cellules tumorales, bien que cet antigène ne soit pas détectable à leur surface par les méthodes immunologiques traditionnelles.

On entend ici par toxine, toute molécule ne comprenant pas de site de fixation non spécifique ou en étant débarrassée, suffisamment toxique pour que, aux doses que l'on peut faire pénétrer dans les cellules au moyen de l'immunotoxine selon l'invention, elle soit suffisamment toxique pour les tuer. Avantageusement la toxine choisie peut être d'origine végétale ou bactérienne comme par exemple la chaîne A de la ricine, la gélonine, l'abrine ou la chaîne A de la toxine diphtérique.

L'anticorps et la toxine peuvent être associés par tout moyen compatible avec leurs propriétés respectives, notamment leur structure, et avec leur utilisation en thérapeutique ; l'invention concerne également les procédés de préparation de l'immunotoxine définie ci-dessus.

Selon un mode préféré de réalisation de l'invention, l'anticorps et la toxine sont associés par couplage par l'intermédiaire d'au moins une liaison covalente. Dans ce cas le procédé de préparation implique de préférence une réaction de condensation entre des fonctions réactives complémentaires portées par l'anticorps monoclonal d'une part et la toxine d'autre part, en présence d'un agent de condensation choisi selon les natures respectives de ces fonctions.

Par exemple, les agents de condensation peuvent être constitués par des carbodiimides lorsque les fonctions complémentaires sont respectivement constituées par des groupes carboxyle et amine, ou vice versa, par exemple le p-toluène-sulfonate de N-cyclohexyl-N'-(β-(N-méthyl-morpholino)-éthyl)-carbodiimide et le chlorhydrate de

(3-méthyl)-aminopropyl-carbodiimide ou le dicyclohexyl-carbodiimide. La réaction est alors conduite dans des conditions usuelles dans la chimie des protéines.

Les agents de condensation peuvent encore être constitués par un 6-maléimido-caproïque-acyl-N-hydroxy-succinimide-ester ou le N-succinimidyl-3-(2-pyridyl-dithio)-propionate (SPDP), lorsque les fonctions complémentaires sont respectivement, au départ, un groupe sulfhydryle et un groupe amine ou vice versa.

Les conditions de réaction sont notamment conformes à celles qui sont indiquées dans les articles auxquels référence est faite dans les exemples qui suivent.

On peut également avoir recours aux techniques décrites dans la demande de brevet EP 0 063 988.

Selon un autre mode de réalisation de l'invention l'anticorps monoclonal et la toxine sont associés par l'intermédiaire d'un liposome. Plus précisément, la toxine choisie est alors "encapsulée" dans des liposomes qui sont eux-mêmes associés, notamment au moyen d'un couplage par liaison(s) covalente(s) par exemple par l'intermédiaire du SPDP, avec l'anticorps monoclonal choisi /voir notamment J. BIOL. Chem. 255, 8015-8018 (1980) ; Nature, vol 293, n° 5829, page 226-8, (1981) ; et Journal of Supramolecular Structure and Cellular Biochemistry, 16, p. 243-258 (1981)/.

L'anticorps monoclonal spécifique peut être obtenu selon les différentes techniques classiques d'obtention d'anticorps monoclonaux à partir d'un hybridome obtenu par fusion de cellules spléniques présentant l'information génétique nécessaire pour produire l'anticorps spécifique et de cellules myélomateuses adaptées à la croissance continue et vigoureuse en culture, avec sélection subséquente du clone recherché, selon des techniques elles-mêmes classiques.

Selon un mode de réalisation de l'invention, les cellules spléniques utilisées sont obtenues à partir d'un animal, par exemple un rat, immunisé par des cellules malignes porteuses de l'antigène CTH, comme par

exemple les cellules de BURKITT [voir par exemple WIELS J. et coll., Proc. Natl. Acad. Sci. USA 78 : 6485-8 (1981)].

Selon un autre mode de réalisation de l'invention qui dans certains cas peut se révéler plus avantageux car il devrait faciliter la sélection de l'hybridome produisant l'anticorps recherché, on immunise l'animal non pas au moyen de cellules malignes porteuses de l'antigène CTH mais au moyen d'un immunogène obtenu par association, notamment par couplage, du CTH avec une molécule de poids moléculaire élevé ou un support macromoléculaire.

Le CTH peut être isolé à partir de cellules animales ou encore synthétisé [voir par exemple l'article de E. NUDELMAN et Coll. dans Science, 220, pages 508-511 (1983)].

A titre d'exemples de molécules porteuses ou supports macromoléculaires pouvant être utilisés on mentionnera des protéines naturelles telles que l'anatoxine tétanique, l'ovalbumine, des sérums albumines, des hémocyanines et analogues.

A titre d'exemples de supports macromoléculaires synthétiques on mentionnera entre autres des polylysines ou des poly(D,L-alanine)-poly(L-lysine).

La littérature mentionne encore d'autres types de supports macromoléculaires susceptibles d'être utilisés ; ils présentent en général un poids moléculaire supérieur à 20 000.

L'association du CTH avec le support macromoléculaire choisi peut être réalisée selon des procédés connus en soi, tels que par exemple celui décrit par FRANTZ et ROBERTSON dans Infect. and Immunity, 33, 193-198 (1981), ou celui décrit par P.E. KAUFFMAN dans Applied and Environmental Microbiology, octobre 1981, vol 42, n° 4, pages 611-4.

Selon un mode de réalisation avantageux et préféré de l'invention, l'anticorps monoclonal entrant dans la

constitution de l'immunotoxine selon l'invention est l'anticorps monoclonal 38-13

produit par l'hybridome déposé dans la "Collection Nationale de microorganismes" de l'Institut Pasteur, 28, rue du Docteur Roux, 75724 PARIS CEDEX 15 (France) le 24 juin 1983, sous le n° I 227. L'obtention de cet anticorps est décrite notamment par J. WIELS et Coll. dans Proc. Natl. Acad. Sci. USA 78 : 6485-8 (1981).

Selon une forme particulièrement préférée de réalisation de l'invention, on associe l'anticorps monoclonal 38-13 à la chaîne A de la ricine, notamment au moyen d'un couplage par covalence, pour obtenir l'immunotoxine appelée "38-13-ch-A-ricine".

Selon une autre forme particulièrement préférée de réalisation de l'invention, on associe l'anticorps monoclonal 38-13 à la gélonine, notamment au moyen d'un couplage par covalence, pour obtenir l'immunotoxine appelée "38-13-gélonine".

La préparation des immunotoxines 38-13-ch-A-ricine et 38-13-gélonine ainsi que leur étude pharmacologique in vitro d'une part et in vivo d'autre part sont décrites dans les exemples donnés plus loin.

Les immunotoxines selon l'invention sont hautement spécifiques et toxiques in vitro et in vivo pour les cellules malignes portant l'antigène CTH.

Leur toxicité se manifeste très rapidement, l'inhibition de 50 % de la synthèse des protéines étant atteinte au bout d'un temps de l'ordre de 10 fois inférieur à celui requis par les immunotoxines connues, ceci en raison d'une internalisation extrêmement rapide de la toxine après fixation de l'immunotoxine sur la cellule. On peut à ce propos émettre l'hypothèse que la toxicité particulièrement élevée des immunotoxines selon l'invention vis-à-vis des cellules malignes, c'est-à-dire leur effet thérapeutique favorable, est due à cette internalisation extrêmement rapide qui réduit largement les risques de dégradation de l'immunotoxine *in vivo*, avant qu'elle n'atteigne les cellules-cibles.

De plus, et c'est là une caractéristique très surprenante des immunotoxines selon l'invention, elles sont capables d'inhiber la synthèse des protéines dans des cellules de lignées tumorales apparemment non reconnues par l'anticorps 38-13 lorsque les techniques immunologiques classiques sont utilisées (par exemple cytotoxicité dépendante du complément et immunofluorescence indirecte mesurée par cytofluorographie), et de tuer ces cellules.

L'immunotoxine selon l'invention peut être utilisée pour la destruction *in vivo* et *in vitro* de certaines cellules malignes, telles que notamment les cellules de BURKITT de types RAMOS, DAUDI, P3HR1, RAJI définies par J. WIELS et Coll. dans Int. J. Cancer 29, 653-658, 1982 et d'autres cellules tumorales ou transformées humaines portant l'antigène CTH en petites quantités telles que les lignées humaines suivantes : K562 (leucémie myéloïde chronique) définie par J. WIELS et Coll. dans Int. J. Cancer 29, 653-658, (1982), Priess, IGROV (lignée humaine provenant d'une tumeur ovarienne) et MOLT-4 (leucémie T).

L'utilisation *in vivo* peut se faire par voie intraveineuse ou locale. La dose à utiliser dépend essentiellement du sujet à traiter et de son état pathologique. A titre indicatif, l'immunotoxine peut être utilisée par voie i.v. à raison de 5 à 100 mg par jour.

L'immunotoxine selon l'invention peut avantageusement être utilisée *in vitro*, à des concentrations notamment de l'ordre de $10^{-8}$ à $10^{-7}$ M, avec une durée d'incubation très courte (de l'ordre de 30 minutes) notamment pour débarrasser des matières biologiques telles qu'une moelle osseuse, de cellules malignes avant

réinjection par autotransplantation à un patient ayant subi une irradiation totale.

Les doses efficaces in vivo sont très inférieures aux doses toxiques (essai chez la souris), ce qui permet de conclure que l'immunotoxine selon l'invention présente un très bon indice thérapeutique.

L'invention sera mieux comprise grâce aux exemples non limitatifs qui suivent.

Dans ces exemples on donnera, à titre illustratif la préparation de deux immunotoxines selon l'invention comprenant l'anticorps monoclonal 38-13 et les compte rendus d'essais pharmacologiques effectués in vitro d'une part et in vivo d'autre part avec ces immunotoxines.

On rappellera d'abord brièvement la préparation et les principales propriétés de cet anticorps monoclonal qui sont décrites dans la littérature.

Rappel : préparation et principales propriétés de l'anticorps monoclonal 38-13 :

Cet anticorps qui est une IgM de rat a été produit par hybridation entre, d'une part des lymphocytes spléniques de rats LEWIS immunisés par des cellules de la lignée de BURKITT africain DAUDI présentant l'antigène CTH et d'autre part des cellules du plasmocytome murin SP20/Ag14. Sa spécificité anti-BURKITT a été démontrée par micro-cytotoxicité dépendante du complément et immunofluorescence indirecte (J. WIELS et Coll. Proc. Nat. Acad. Sci., USA, 1981, 78, 6485-6488). Sur une série de lignées tumorales humaines établies en culture, l'anticorps monoclonal 38-13 réagit avec les lignées provenant de tumeurs de BURKITT, qu'elles contiennent ou non le génome du virus d'EPSTEIN-BARR, et ne réagit avec aucune autre lignée tumorale humaine. En particulier, l'anticorps 38-13 ne reconnaît pas les cellules des lignées lymphoblastoïdes établies par infection in vitro de lymphocytes B normaux par le virus d'EPSTEIN-BARR (J. WIELS, et Coll. Int. J. Cancer, 1982, 29, 653-658). L'antigène reconnu par l'anticorps 38-13 à la surface des cellules de BURKITT est un glycolipide neutre alors que les anticorps utilisés dans les immunotoxines étudiées jusqu'à présent reconnaissaient des protéines se trouvant à la surface des cellules, et l'anticorps réagit vraisemblablement avec la partie glucidique de cet antigène, puisque la présence de galactose à forte concentration (0,1M) dans le milieu inhibe la fixation de l'anticorps sur les cellules cibles (M. LIPINSKI, et Coll., J. Immunol. 1982, 129, 2301-2304). Ce glycolipide neutre est le globotriaosylcéramide, ou céramidetrihexosyle (CTH) de structure indiquée plus haut (E. NUDELMAN et Coll., Science, 1983, 220, 508-511).

L'anticorps 38-13 montre une spécificité stérique stricte pour cet antigène puisqu'il ne réagit pas avec l'isomère positionnel : Gal$\alpha$(1⟶3), Gal... Le CTH a la même structure que la substance Pk, précurseur de l'antigène du groupe sanguin P. L'accumulation de cette substance intermédiaire Pk sur certaines cellules malignes telles que les cellules de BURKITT pourrait être liée à des anomalies du fonctionnement de certaines enzymes de la chaîne de synthèse de la substance P dans ces cellules, du même type que les anomalies enzymatiques rapportées dans divers systèmes tumoraux expérimentaux.

Exemple 1

Immunotoxine 38-13-ch-A-ricine

La ricine est une toxine d'origine végétale qui est constituée d'un polypeptide toxique (chaîne A) lié à un polypeptide se fixant sans spécificité sur les cellules (chaîne B) (voir par exemple E.S. VITETTA et Coll., Science, 249, p. 644-650, 1983).

Dans cet exemple, la ricine est extraite et purifiée à partir de graines de *Ricinus communis* selon la technique décrite par LUGNIER et Coll., C.R. Acad. Sci. Paris 273 : 704-707, 1971. La séparation des chaînes A et B est effectuée selon la méthode décrite par F.K. JANSEN et Coll., Immunol. Rev. 62 : 185-216 ; 1982.

L'anticorps monoclonal 38-13 est purifié par précipitation avec du sulfate d'ammonium à 50 % et filtration sur une colonne de Sépharose 6B (dénomination commerciale) dans du tampon au phosphate de sodium (10 mM, pH 8,0, NaCl 0,5 M, $NaN_3$ à 0,02 %).

La chaîne A de la ricine est couplée avec l'anticorps monoclonal 38-13 en utilisant le 3-(2-pyridyldithio)propionate de N-succinimidyle (SPDP) en tant qu'agent de couplage hétérobifonctionnel, selon la méthode décrite par J. CARLSSON et Coll. dans Biochem J. 173, 727-737 ; (1978).

Le nombre de molécules de chaîne A de la ricine couplées par IgM est déterminé par mesure de la libération de 2-thiopyridone après réduction (T. STUCHBURY, Biochem. J. 151, 417-432, (1975)). L'immunotoxine 38-13-ch-A-ricine contient environ 10 molécules de chaîne A de la ricine par IgM.

Exemple 2

Immunotoxine 38-13-gélonine

La gélonine est préparée à partir de graines de *Geloninum multiflorum* et purifiée selon la méthode décrite par F. STIRPE et Coll. dans J. Biol. Chem. 255, 6947-6953, (1980).

L'anticorps monoclonal 38-13 est purifié comme dans l'exemple 1 et couplé avec la gélonine en utilisant le SPDP comme agent de couplage, selon la méthode décrite par P.E. THORPE et Coll. dans Europ. J. Biochem, 116, 447-454, (1981).

En utilisant la même méthode de détermination que dans l'exemple 1, on constate que l'immunotoxine obtenue contient environ 10 molécules de gélonine par IgM.

Essais in vitro :

Les immunotoxines 38-13-ch-A-ricine et 38-13-gélonine ont été utilisées dans divers systèmes expérimentaux. Ces immunotoxines se sont montrées hautement toxiques pour les cellules de la lignée de BURKITT RAMOS.

L'immunotoxine 38-13-ch-A-ricine est capable d'inhiber la synthèse des protéines (mesurée par incorporation d'acides aminés marqués), à une concentration 5 000 fois plus petite que celle de la chaîne A de la ricine purifiée. Elle se révèle donc pratiquement aussi toxique sur les cellules RAMOS que la ricine entière (inhibition de 50 % de la synthèse des protéines obtenue pour des concentrations de $10^{-6}$ M pour la chaîne A purifiée, de $5.10^{-10}$ M pour l'immunotoxine 38-13-ch-A-ricine et pour la ricine entière). Cette activité cytotoxique de l'immunotoxine a deux caractéristiques originales :

- elle est extrêmement rapide, puisqu'une étude cinétique montre que l'inhibition de 50 % de la synthèse des protéines est atteinte dans les cellules RAMOS après un temps d'incubation de 2 h à 2h30, ce qui est pratiquement comparable à la cinétique d'action de la ricine entière. On peut rappeler que pour toutes les immunotoxines décrites précédemment, les temps d'incubation minimaux pour observer un effet toxique varient de 16 h à 30 h. Cette rapidité d'action semble être liée à une internalisation extrêmement rapide de l'immunotoxine après fixation sur la cellule.

L'immunotoxine 38-13-gélonine inhibe également très fortement la synthèse des protéines dans les cellules RAMOS, et se révèle 1000 fois plus active que la gélonine non couplée.

De même que la fixation de l'anticorps 38-13 non couplé, l'action des immunotoxines décrites ici est inhibable de façon simple par le galactose. L'addition de 0,1 M de D-galactose dans le milieu de cinétique suffit à inhiber l'effet toxique des conjugués.

Enfin, ces immunotoxines sont capables d'inhiber la synthèse des protéines et de tuer des cellules de lignées tumorales apparemment non reconnues par l'anticorps 38-13 lorsque les techniques immunologiques classiques sont utilisées (cytotoxicité dépendante du complément, immunofluorescence indirecte mesurée par cytofluorographie). Ainsi, ces immunotoxines se révèlent toxiques pour la lignée lymphoblastoïde Priess, qui contient le virus d'EPSTEIN BARR, mais n'est pas une cellule de BURKITT, pour les lignées leucémiques humaines K562 et la leucémie murine L1210. Cette toxicité est vraisemblablement liée à la fixation des immunotoxines sur des sites antigéniques reconnus par l'anticorps 38-13, en nombre insuffisant pour être révélés par les méthodes immunologiques standards, mais en nombre suffisant pour permettre l'entrée de quelques molécules d'immunotoxines dans la cellule. En effet, la toxicité des immunotoxines dans ces lignées apparemment totalement distinctes au plan phylogénétique, est également inhibable par le galactose à la concentration de 0,1 M comme l'est la fixation de l'anticorps 38-13 sur ces cibles spécifiques. De plus, une immunotoxine contenant un anticorps monoclonal non pertinent, IgM 13.6 anti-TNP, préparée suivant le même procédé de couplage biochimique, purifiée de la même façon et contenant environ 10 molécules de chaîne A de la ricine par IgM, ne présente aucune effet toxique sur les cellules qui ne possèdent pas l'haptène TNP.

En conclusion, les immunotoxines selon l'invention se révèlent actives <u>in vitro</u> contre des cellules tumorales d'origines diverses.

De façon générale, on a constaté que les immunotoxines 38-13-ch-A-ricine et 38-13-gélonine sont capables de tuer les cellules de BURKITT portant l'antigène CTH en grandes quantités [lignées RAMOS, DAUDI, P3HR1, RAJI définies par J. WIELS et Coll. dans Int. J. Cancer 29, 653-658, (1982)], à des concentrations de l'ordre de $10^{-8}$ M et d'autres cellules tumorales ou transformées humaines suivantes : K562++ (leucémie myéloïde chronique), Priess, IGROV (une lignée humaine provenant d'une tumeur ovarienne), MOLT-4 (leucémie T). Toutes ces cellules sont tuées par l'immunotoxine selon l'invention à des concentrations égales ou inférieures à $10^{-8}$ M.

Etude in vivo

La cellule leucémique murine L1210 s'étant révélée sensible in vitro aux immunotoxines 38-13-ch-A-ricine et 38-13-gélonine dans la même gamme de concentrations, un essai de l'activité thérapeutique de cette immunotoxine in vivo a pu être réalisé. Cette leucémie est une tumeur expérimentale très résistante à la plupart des substances antitumorales. Elle est utilisée pour la sélection des agents susceptibles d'être employés en clinique humaine, car il existe une très bonne corrélation entre activité en clinique humaine et activité sur ce système expérimental (voir J.M. VENDITTI, "Relevance of transplantable animal tumor systems to the selection of new agents for clinical trial in pharmacological basis of cancer chemotherapy". The university of Texas ed. WILLIAMS and WILKINS, publ. 1975 Baltimore USA). D'autre part, la tumeur expérimentale qu'est la leucémie L1210 de la souris est en effet couramment utilisée pour l'évaluation de tous les composés antitumoraux actuellement mis en oeuvre en thérapeutique humaine, ainsi qu'il est décrit, par exemple par C.C. ZUBROD dans Proc. Nat. Acad. Sci. USA, 69, 1972 p. 1042-1047. Le système tumoral ainsi constitué expérimentalement permet une évaluation quantitative très précise de l'activité du composé testé et, par conséquent aussi, une comparaison objective entre les activités respectives de différents composés, par exemple selon les méthodes décrites par R.E. SKIPPER et Coll. dans Cancer Chemother. Rep. 35, 1964, p. 1-111 et 45, 1965, p. 5-28.

Une autre tumeur ayant une bonne valeur expérimentale est la tumeur murine de poumon de LEWIS (voir article de J. VENDITTI et Coll. cité ci-dessus).

En pratique deux types de test sont effectués :

1) Test in vitro - in vivo

On incube in vitro 30 minutes les cellules tumorales (LEWIS ou L1210).

Pour les cellules LEWIS la concentration en immunotoxine est 0,5 x $10^{-7}$ M pour 2 x $10^{6}$ cellules dans 0,2 ml.

Pour les cellules L1210 la concentration en immunotoxine est 0,5 x $10^{-8}$ M pour $10^{5}$ cellules dans 0,2 ml.

Les cellules témoins sont incubées dans les mêmes conditions en absence d'immunotoxines.

Les cellules sont ensuite injectées à des lots de 10 souris (C57 Black pour les cellules LEWIS et DBA/2 pour les cellules L1210). La survie des animaux est déterminée. Dans le cas où les cellules tumorales sont tuées par le traitement in vitro, les animaux ayant reçu l'injection ne développent pas de tumeur et survivent, alors que les animaux témoins meurent tous après un délai dépendant du nombre de cellules tumorales injectées.

Dans cette expérience les animaux témoins ayant reçu une injection de cellules LEWIS sont morts au bout de 20,5 $\pm$ 0,7 jours. Tous les animaux ayant reçu les cellules incubées avec l'immunotoxine étaient vivants après 45 jours.

Pour les cellules L1210, les animaux témoins ayant reçu une injection de cellules L1210 sont morts au bout de 9,0 $\pm$ 0,26 jours. Tous les animaux ayant reçu les cellules incubées avec l'immunotoxine étaient vivants après 45 jours.

On a ainsi démontré qu'un grand nombre de cellules tumorales ($10^{5}$ à $10^{6}$) peuvent être tuées in vitro après une incubation très courte (30 minutes) avec l'immunotoxine selon l'invention. Cette immunotoxine peut donc être utilisée pour détruire in vitro les cellules tumorales se trouvant dans une moelle osseuse devant être greffée par autotransplantation à un patient ayant subi une irradiation totale ou une chimiothérapie massive.

2) Test in vivo

On utilise des souris ayant reçu par voie intrapéritonéale un inoculum de cellules de leucémie L1210. La moitié d'entre elles sont soumises 1 heure après ladite inoculation tumorale à une injection intrapéritonéale d'une dose unique de l'immunotoxine à tester : l'autre moitié des souris reçoit une injection d'un volume identique d'un solvant inactif et sert de série témoin. Les souris sont réparties au hasard dans chaque série expérimentale.

Une première expérimentation de routine permet de déterminer la mortalité par toxicité et de fixer ainsi les doses infra-létales.

Conformément à la convention en usage dans ce domaine, on considère comme guéris les animaux qui survivent plus de 45 jours après l'inoculation de cellules tumorales.

On peut ainsi déterminer la mortalité par toxicité sous l'effet d'une injection unique de l'un des composés à différentes doses (doses infra-létales, DL 50 et DL 100) et évaluer le taux de survie du groupe d'animaux auxquels on n'a injecté qu'une dose infra-létale du composé à tester.

On calcule le taux de survie à partir d'une analyse statistique des résultats expérimentaux, par comparaison avec les résultats obtenus avec la série témoin, selon les méthodes bien connues de MANN-WHITEY et de WILCOXON ; le pourcentage de cellules leucémiques tuées par le traitement réalisé est calculé par la méthode décrite par H.E. SKIPPER et Coll. dans les articles cités plus haut, en prenant pour base, suivant le cas, soit l'augmentation de la survie moyenne en l'absence de survivants, soit le pourcentage de survivants.

Résultats obtenus :

| Dose d'immunotoxine | Survie moyenne en jours des souris témoins ± erreur standard | Survie moyenne en jours des souris traitées ± erreur standard | % de cellules tumorales tuées par le traitement |
|---|---|---|---|
| 50µg/souris | 9,0 ± 0,25 | 12,8 ± 0,36 | 99,9 |
| 100µg/souris | 9,0 ± 0,25 | 12,6 ± 0,37 | 99,9 |

La dose maximum tolérée d'immunotoxine est de 500 µg par souris.

Cette pluralité de résultats est tout à fait remarquable. L'immunotoxine selon l'invention présente un spectre d'action tumoricide *in vitro* très étendu et ce tant à l'égard des cellules tumorales portant à leur surface l'antigène susdéfini en quantité suffisante pour être décelable en tant que tel par les méthodes fines d'analyse immunologique pratiquées à ce jour, qu'à l'égard des cellules tumorales chez lesquelles cet antigène paraît présent mais en proportion non décelable par les susdites méthodes fines d'analyse ; cette activité est tout autant remarquable <u>in vivo</u>, comme il résulte de ce qui précède. L'immunotoxine selon l'invention présente donc un large spectre d'activité anti-tumorale, puisque l'action tumoricide de l'immunotoxine selon l'invention s'exerce à l'égard de cellules tumorales aussi éloignées génétiquement que les cellules de lymphosarcome de BURKITT d'origine humaine que contre des cellules de tumeur L1210 de la souris. Enfin, le pouvoir discriminant qu'exerce l'immunotoxine entre les cellules tumorales et les cellules saines est considérable, si l'on tient compte de la large gamme de doses d'immunotoxine qui peuvent être utilisées <u>in vivo</u> sans risque de toxicité pour l'hôte vivant.

REVENDICATIONS

1. Immunotoxine caractérisée en ce qu'elle comprend essentiellement un anticorps de préférence monoclonal, spécifique du globotriaosylcéramide, ou céramidetrihexosyle, de structure:

Galα(1——>4)Galβ(1——>4)Glcβ(1——>1) céramide,

associé avec une toxine, dans des conditions permettant l'internalisation de la toxine dans les cellules animales.

2. Immunotoxine selon la revendication 1, caractérisée en ce que l'anticorps et la toxine sont associés par couplage par l'intermédiaire d'au moins une liaison covalente.

3. Immunotoxine selon la revendication 1 ou 2, caractérisée en ce que l'anticorps spécifique est l'anticorps 38-13 produit par l'hybridome déposé sous le n° I 227 dans la "Collection Nationale de microorganismes" tenue par l'Institut Pasteur à Paris.

4. Immunotoxine selon l'une des revendications 1 à 3, caractérisée en ce que la toxine est la chaîne A de la ricine ou la gélonine.

5. Immunotoxine résultant de l'association, notamment au moyen d'un couplage par covalence, de l'anticorps monoclonal 38-13 avec la chaîne A de la ricine.

6. Immunotoxine résultant de l'association, notamment au moyen d'un couplage par covalence, de l'anticorps monoclonal 38-13 avec la gélonine.

7. Composition pharmaceutique, caractérisée en ce qu'elle comprend, en tant que substance active, au moins une immunotoxine selon l'une quelconque des revendications 1 à 6, en association avec un véhicule pharmaceutique.

8. Immunotoxine selon l'une quelconque des revendications 1 à 6 pour l'utilisation pour la prévention ou la destruction des cellules tumorales in vivo.

9. Procédé pour débarrasser in vitro une matière biologique de cellules malignes, caractérisé en ce qu'il

consiste à faire incuber ladite matière avec l'immunotoxine selon l'une quelconque des revendications 1 à 6.

0130132

Office européen des brevets

**RAPPORT DE RECHERCHE EUROPEENNE**

Numéro de la demande

EP 84 40 1369

## DOCUMENTS CONSIDERES COMME PERTINENTS

| Catégorie | Citation du document avec indication, en cas de besoin des parties pertinentes | Revendication concernée | CLASSEMENT DE LA DEMANDE (Int. Cl. 3) |
|---|---|---|---|
| X,P | CHEMICAL ABSTRACTS, vol. 100, no. 11, 12 mars 1984, page 404, no. 83927q, Columbus, Ohio, US; J. WIELS et al.: "Properties of immunotoxins against a glycolipid antigen associated with Burkitt's lymphoma" & CANCER RES. 1984, 44(1), 129-33 * abrégé * --- | 1-9 | A 61 K 39/44<br>A 61 K 39/395 |
| Y | CHEMICAL ABSTRACTS, vol. 98, no. 21, 23 mai 1983, page 45, no. 172716c, Columbus, Ohio, US; T. TAKAHASHI et al.: "Monoclonal anti-MM46 antibody. Ricin A chain conjugate. In vitro and in vivo antitumor activity" & GAN TO KAGAKU RYOHO 1983, 10(2,Pt.2), 544-50 * abrégé * --- | 1,2,4,5,7-9 | |
| | | | DOMAINES TECHNIQUES RECHERCHES (Int. Cl. 3) |
| Y | CHEMICAL ABSTRACTS, vol. 99, no. 2, 11 juillet 1983, page 300, no. 10769r, Columbus, Ohio, US; L.V. LEVIN et al.: "Selective cytotoxicity for a colorectal carcinoma cell line by a monoclonal anti-carcinoembryonic antigen antibody coupled to the A chain of ricin" & J. BIOL. RESPONSE MODIF. 1982, 1(2), 149-62 * abrégé * --- -/- | 1,2,4,5,7-9 | A 61 K |

Le présent rapport de recherche a été établi pour toutes les revendications

| Lieu de la recherche | Date d'achèvement de la recherche | Examinateur |
|---|---|---|
| LA HAYE | 01-10-1984 | REMPP G.L.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

## DOCUMENTS CONSIDERES COMME PERTINENTS

| Catégorie | Citation du document avec indication, en cas de besoin des parties pertinentes | Revendication concernée | CLASSEMENT DE LA DEMANDE (Int. Cl. 3) |
|---|---|---|---|
| Y,D | BIOLOGICAL ABSTRACTS, vol. 71, no. 3, 1981, page 2184, no. 20870, Philadelphia, P.A., US; F. STIRPE et al.: "Gelonin, a new inhibitor of protein synthesis, nontoxic to intact cells: isolation, characterization and preparation of cytotoxic complexes with concanavalin A" & J. BIOL. CHEM. 255(14): 6947-6953, 1980 * abrégé * | 1,2,4, 6-9 | |
| | --- | | |
| Y,D | BIOLOGICAL ABSTRACTS, vol. 73, no. 5, 1982, page 3434, no. 33350, Philadelphia, P.A., US; P.E. THORPE et al.: "Cytotoxicity acquired by a conjugation of an anti-THy 1.1 monoclonal antibody and the ribosome-inactivating protein, gelonin" & EUR. J. BIOCHEM. 116(3): 447-454, 1981 * abrégé * | 1,2,4, 6-9 | DOMAINES TECHNIQUES RECHERCHES (Int. Cl. 3) |
| | --- | | |
| Y | BIOLOGICAL ABSTRACTS, vol. 74, no. 2, 1982, page 1205, no. 11610, Philadelphia, P.A., US; VIC RASO et al.: "Monoclonal antibody-ricin A chain conjugate selectively cytotoxic for cells bearing the common acute lymphoblastic leukemia antigen" & CANCER RES. 42(2): 457-464, 1982 * abrégé * | 1,2,4, 5,7-9 | |
| | --- -/- | | |

Le présent rapport de recherche a été établi pour toutes les revendications

| Lieu de la recherche | Date d'achèvement de la recherche | Examinateur |
|---|---|---|
| LA HAYE | 01-10-1984 | REMPP G.L.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

Office européen des brevets

**0130132**

Numéro de la demande

# RAPPORT DE RECHERCHE EUROPEENNE

EP 84 40 1369

Page 3

## DOCUMENTS CONSIDERES COMME PERTINENTS

| Catégorie | Citation du document avec indication, en cas de besoin des parties pertinentes | Revendication concernée | CLASSEMENT DE LA DEMANDE (Int. Cl. 3) |
|---|---|---|---|
| Y | NATURE, vol. 294, 12 novembre 1981, pages 171-173, Macmillan Journals Ltd.; I.S. TROWBRIDGE et al.: "Anti-transferrin receptor monoclonal antibody and toxin-antibody conjugates affect growth of human tumour cells" * article en entier * | 1,2,4, 5,7-9 | |
| Y | FR-A-2 466 252 (CM INDUSTRIES) * page 1, ligne 29 - page 2, ligne 9 * | 1,2,4, 5,7,9 | |
| Y | US-A-4 356 117 (DAVID M. NEVILLE Jr. et al.) * colonne 3, ligne 25 - colonne 4, ligne 17 * | 1,2,4, 5,7-9 | DOMAINES TECHNIQUES RECHERCHES (Int. Cl. 3) |
| Y | EP-A-0 023 401 (TEIJIN LTD.) * page 4, lignes 2-29; page 5, ligne 19 - page 15, ligne 2 * | 1,2,4, 5,7-9 | |
| Y | EP-A-0 044 167 (THE REGENTS OF THE UNIVERSITY OF CALIFORNIA) * page 1, ligne 6 - page 6, ligne 27 * | 1,2,4-9 | |
| Y,D | EP-A-0 063 988 (SANOFI) * page 1, ligne 1 - page 4, ligne 33 * | 1,2,4, 5,7-9 | |

--- -/-

Le présent rapport de recherche a été établi pour toutes les revendications

| Lieu de la recherche | Date d'achèvement de la recherche | Examinateur |
|---|---|---|
| LA HAYE | 01-10-1984 | REMPP G.L.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82

Office européen des brevets

**RAPPORT DE RECHERCHE EUROPEENNE**

0130132

Numéro de la demande

EP 84 40 1369

Page 4

| DOCUMENTS CONSIDERES COMME PERTINENTS | | | |
|---|---|---|---|
| Catégorie | Citation du document avec indication, en cas de besoin des parties pertinentes | Revendication concernée | CLASSEMENT DE LA DEMANDE (Int. Cl. 3) |
| Y | EP-A-0 080 401 (SANOFI)<br><br>* page 1, ligne 1 - page 6, ligne 1 *<br><br>----- | 1,2,4,5,7-9 | |
| | | | DOMAINES TECHNIQUES RECHERCHES (Int. Cl. 3) |
| | | | |

Le présent rapport de recherche a été établi pour toutes les revendications

| Lieu de la recherche | Date d'achèvement de la recherche | Examinateur |
|---|---|---|
| LA HAYE | 01-10-1984 | REMPP G.L.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82